# EUROPEAN PATENT APPLICATION

(11) **EP 1 568 318 A1**
(43) Date of publication of application: **31.08.2005**
(21) Application number: 05003918.9
(22) Date of filing: 23.02.2005
(51) Int. Cl.: A61B 5/053

(54) **Living body measurement apparatus**

(30) Priority: 24.02.2004 JP 2004048406
(71) Applicant: TANITA CORPORATION, Tokyo (JP)
(72) Inventor: Shimomura, Miyuki, Tanita Corporation, Itabashi-Ku Tokyo (JP); Koyama, Kazuyasu, Tanita Corporation, Itabashi-Ku Tokyo (JP)
(74) Representative: Grünecker, Kinkeldey, Stockmair & Schwanhäusser Anwaltssozietät

(57) **Abstract**

Disclosed is a living body measurement apparatus (1) comprising: a plurality of electrodes (4a,4b,5a,5b,11,13); a current supplying unit (36); a voltage measurement unit (37); a switching unit (35); a controller unit (38); and a display unit (7), wherein said plurality of electrodes are each contact with each of parts of a living body, said current supplying unit supplies electric current to the electrodes, said voltage measurement unit measures electric voltage on the electrodes, said switching unit switches between the electrodes so that the electrodes to which the current is supplied and on which the voltage is measured are changed, said controller unit controls operation of the current supplying unit, the voltage measurement unit and the switching unit, and said display unit is connected to the controller unit and displays the measurement result. According to the present invention, said apparatus further comprises: a display item selection unit (18) and a part-of-living body selection unit (20), whereby the measurement result selected by those two selection units is displayed on the display unit.

## Description

### Background of the Invention:

### Field of the Invention:

The present invention relates to a living body measurement apparatus adapted for measuring the condition of parts of a living body.

### Prior Art:

There has been known such living body measuring apparatus that numerically displays the measurement result (see e.g. Patent Document 1) or graphically displays the measurement result on a display unit (see e.g. Patent Document 2). Another apparatus has also been known in which the measurement result for each of parts of a living body is numerically displayed and a plurality of measurement results for each part are numerically displayed (see e.g. Patent Document 3).
Patent Document 1: Japanese Patent Laid-Open No. 2001-157671;
Patent Document 2: Japanese Patent Laid-Open No. 2001-190514; and
Patent Document 3: Japanese Patent Laid-Open No. 2001-178693.

The prior art living body measurement apparatus has generally been constructed in such manner that the measurement result is numerically displayed because of reduction in cost of a display unit. Unfortunately, as the number of measurement items displayed increases it becomes difficult to immediately judge the measurement result simply at a glance. Accordingly, another apparatus has been put into practice in which the measurement result is graphically displayed, but, the number of measurement items displayed is smaller. Thereafter, still another apparatus has been provided in which measurement of the living body can be done for each of the parts so that the measurement result for each of the parts and each of measurement items is displayed, in addition to display of a plurality of measurement items for each of the parts. In such apparatus, however, the measurement result is displayed only in numerical format.

As the result, if it is desired to display the measurement result for a plurality of measurement items per each part of the living body then the number of numerical values displayed becomes too increased, which is not easy to understand. Furthermore, if it is desired to graphically display the measurement result then operation of selection of the target graph becomes much complicated.

In view of the above it is an object of the present invention to solve the above-mentioned problems in the prior art and to provide an improved living body measurement apparatus that is very easy for a person under test to operate it and to understand the measurement result displayed thereby.

### Summary of the Invention:

To attain such object the present invention provides a living body measurement apparatus comprising: a plurality of electrodes; a current supplying unit; a voltage measurement unit; a switching unit; a controller unit; and a display unit, wherein said plurality of electrodes are each contact with each of parts of a living body, said current supplying unit supplies electric current to the electrodes, said voltage measurement unit measures electric voltage on the electrodes, said switching unit switches between the electrodes so that the electrodes to which the current is supplied and on which the voltage is measured are changed, said controller unit controls operation of the current supplying unit, the voltage measurement unit and the switching unit, and said display unit is connected to the controller unit and displays the measurement result, said apparatus further comprising: a display item selection unit and a part-of-living body selection unit, whereby the measurement result selected by those two selection units is displayed on the display unit.

According to one embodiment of the present invention the living body measurement apparatus may further comprise an additional selection unit which produces graphical display of the measurement result selected by said two selection units on the display unit.

According to another embodiment of the present invention said measurement result may be at least one of the followings: body weight; body fat rate; muscle mass; bone mass; body water mass; basal metabolism; and visceral fat area.

### Effects of the Invention:

According to the present invention, a living body measurement apparatus comprising: a plurality of electrodes; a current supplying unit; a voltage measurement unit; a switching unit; a controller unit; and a display unit, wherein said plurality of electrodes are each contact with each of parts of a living body, said current supplying unit supplies electric current to the electrodes, said voltage measurement unit measures electric voltage on the electrodes, said switching unit switches between the electrodes so that the electrodes to which the current is supplied and on which the voltage is measured are changed, said controller unit controls operation of the current supplying unit, the voltage measurement unit and the switching unit, and said display unit is connected to the controller unit and displays the measurement result, said apparatus further comprising: a display item selection unit and a part-of-living body selection unit, whereby the measurement result selected by those two selection units is displayed on the display unit. Accordingly, the present invention provides a significant advantage in that a person under test can get any desirable display simply by selecting the item and the part of body that the person under test wants to display.

Furthermore, the apparatus of the present invention may comprise an additional selection unit which produces graphical display of the measurement result selected by said two selection units on the display unit. As the result, the display of desired item for the desired part of body can readily be seen in the graphic format.

### Brief Description of the Drawings:

Now, the present invention will be described in more detail with reference to the accompanying drawings in which:
Fig. 1 is a perspective view of a living body measurement apparatus incorporating the present invention;
Fig. 2 is a detailed view of an operation unit 6 and a display unit 7 in Fig. 1;
Fig. 3 is an electrical block diagram of the body composition meter 1 in Fig. 1;
Figs. 4A, 4B and 4C are flow charts each illustrating process of setting the calendar;
Figs. 5A, 5B, 5C and 5D are flow charts each illustrating registration of personal data for a person under test;
Figs. 6A and 6B are flow charts each illustrating measurement using the body composition meter;
Fig. 7 is a flow chart illustrating process of display;
Figs. 8A and 8B are flow charts each illustrating process of display key interruption;
Figs. 9A and 9B, 10A and 10B, and 11A and 11B are flow charts each illustrating process of numerical display for each of display items and each of parts of body;
Figs. 12A and 12B, 13A and 13B, and 14A and 14B are flow charts each illustrating process of daily graphic display for each of display items and each of parts of body; and
Figs. 15 to 27 show examples of display on the display unit.

### Best Mode for Embodying the Invention:

The present invention relates to a living body measurement apparatus comprising: a plurality of electrodes; a current supplying unit; a voltage measurement unit; a switching unit; a controller unit; and a display unit, wherein said plurality of electrodes are each contact with each of parts of a living body, said current supplying unit supplies electric current to the electrodes, said voltage measurement unit measures electric voltage on the electrodes, said switching unit switches between the electrodes so that the electrodes to which the current is supplied and on which the voltage is measured are changed, said controller unit controls operation of the current supplying unit, the voltage measurement unit and the switching unit, and said display unit is connected to the controller unit and displays the measurement result, said apparatus further comprising: a display item selection unit and a part-of-living body selection unit, whereby the measurement result selected by those two selection units is displayed on the display unit.

The living body measurement apparatus may further comprise an additional selection unit which produces graphical display of the measurement result selected by said two selection units on the display unit.

The measurement result may be at least one of the followings: body weight; body fat rate; muscle mass; bone mass; body water mass; basal metabolism; and visceral fat area.

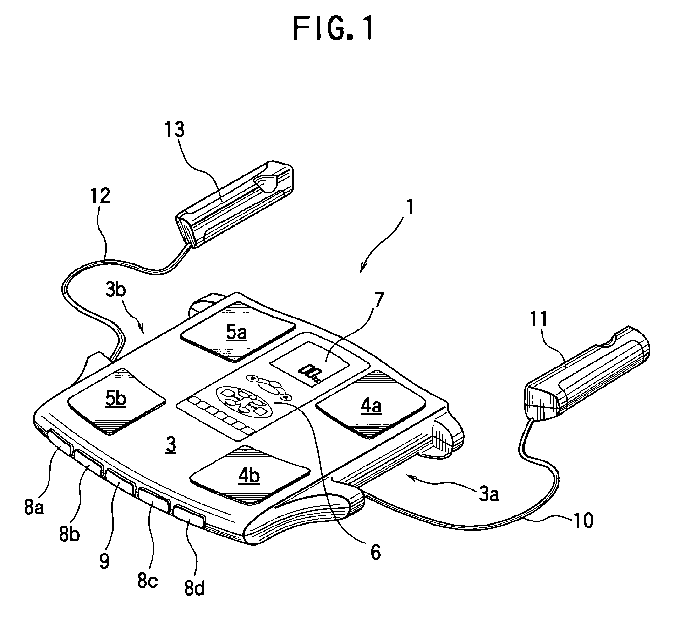

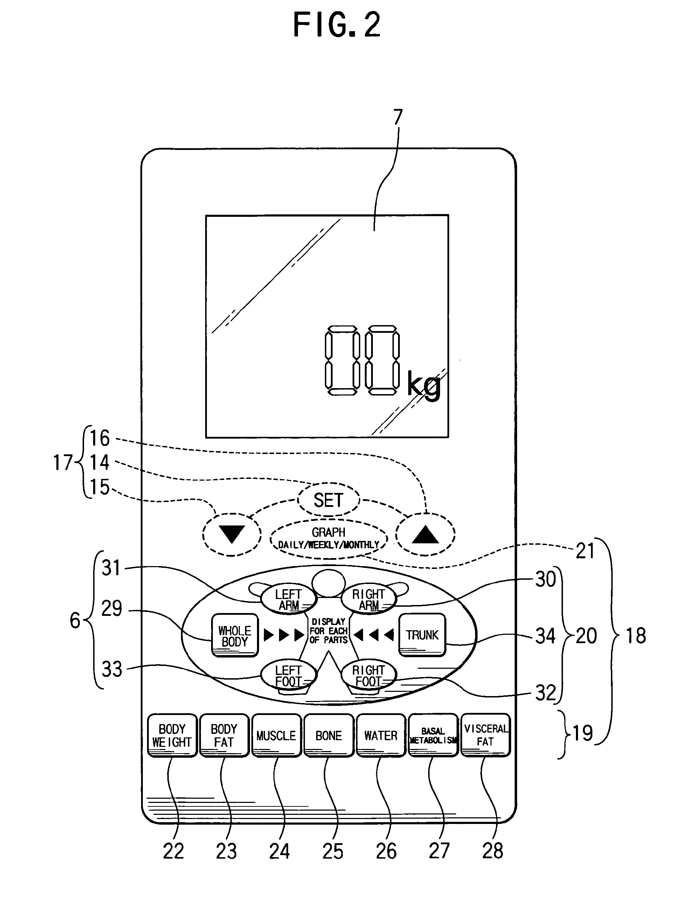

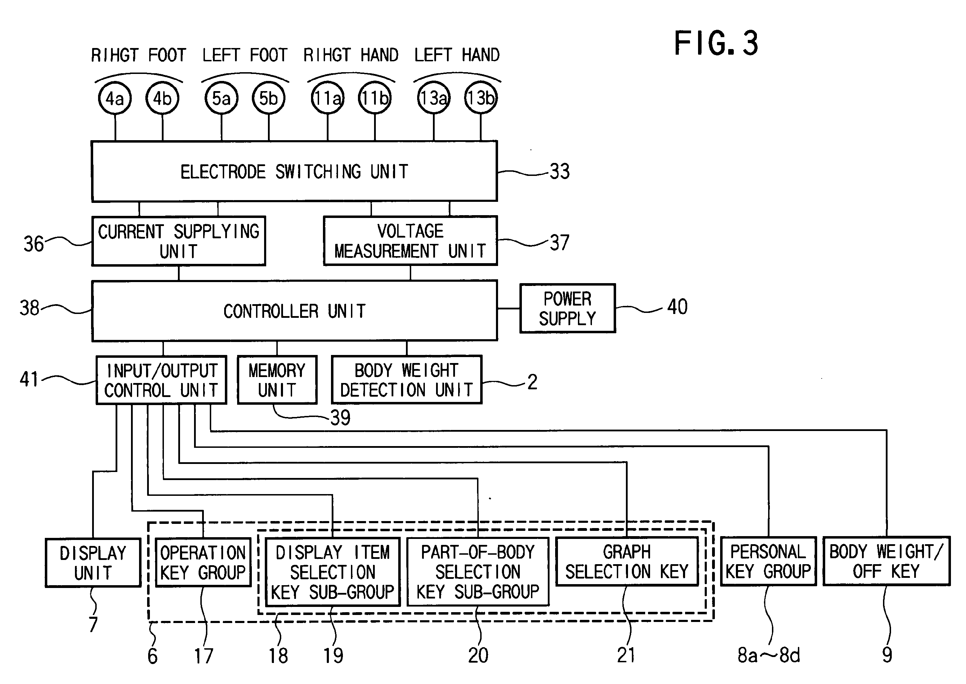

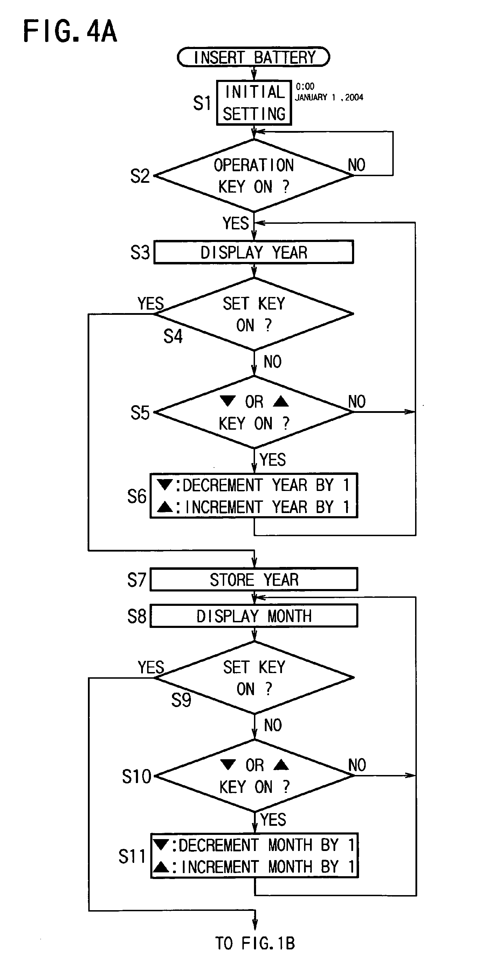

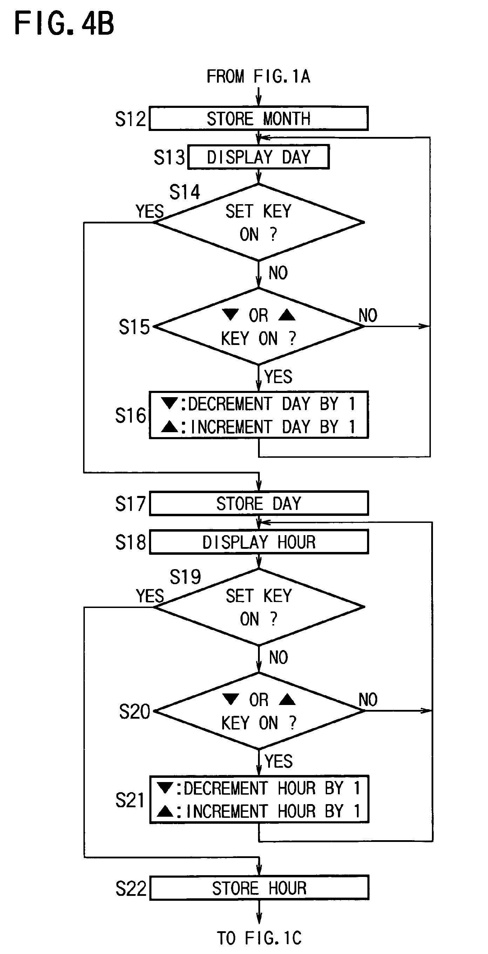

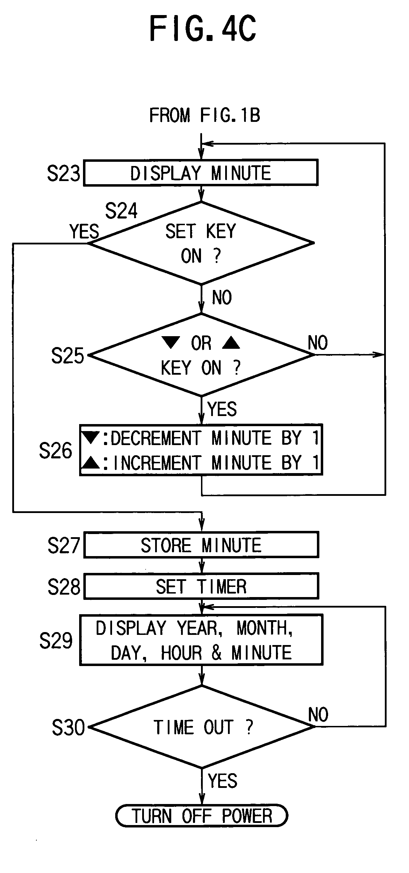

Referring to an embodiment of the present invention, Fig. 1 is a perspective view of a living body measurement apparatus incorporating the present invention, and in particular, a body composition meter 1 using bioelectric impedance method and comprising a plurality of electrodes in contact with eight (8) parts of both hands and both feet of a person under test. The body composition meter 1 has a body weight detection unit 2 included therein for measuring body weight of the person, as with a prior art fat meter for measuring bioelectrical impedance between both feet of the person. It further includes right foot electrodes 4a, 4b, left foot electrodes 5a, 5b, an operation unit 6 for entering various data and for changing display of measurement result, and a display unit 7 for displaying the measurement result, all mounted on top of a platform 3. Mounted also on the platform 3 at the near end thereof are: four (4) personal keys 8a, 8b, 8c and 8d for retrieving the data of the person under test; and a body weight / OFF key 9 for measurement of only body weight and for forcibly turning OFF the power to the apparatus.

The body composition meter 1 further includes a right hand electrode section 11 connected to a circuit board within the apparatus via a cable 10. The right hand electrode 11 is provided with two (2) electrodes 11a and 11b on the outer surface thereof. In the same manner, a left hand electrode section 13 is connected via a cable 12 and it is provided with two (2) electrodes 13a and 13b. The right hand electrode section 11 and the left hand electrode section 13 are constructed to be integral with the platform 3 by mating them with right and left engagement sections 3a and 3b of the platform 3. The body composition meter 1 is configured in the same manner as the eight (8) - electrode body fat meter in the prior art, and therefore, detailed description thereof is omitted here.

Fig. 2 is a detailed view of the operation unit 6 and the display unit 7 in Fig. 1. In particular, as shown in Fig. 2, the display unit 7 made up of a dot matrix LCD (Liquid Crystal Display) for numerical and graphic display is provided at the upper side, whereas the operation unit 6 is provided at the lower side. The operation unit 6 comprises an operation key group 17 including a "SET" key 14 for entering various types of data, a DOWN key 15 for decreasing the numerical data, and an UP key 16 for increasing the numerical data. The operation unit 6 further comprises a display selection key group 18 including a display item selection key sub-group 19 for selecting a display item for the body compositions measured, a part-of-body selection key sub-group 20 for selecting a part of the body to be displayed, and a graph selection key 21 for selecting a graphical display of the numerical data displayed.

The display item selection key sub-group 19 is provided for selecting a display item for the body compositions, and is consisting of a body weight key 22 for displaying body weight value, a body fat key 23 for displaying body fat rate, a muscle key 24 for displaying muscle mass, a bone mass key 25 for displaying bone mass, a body water key 26 for displaying body water mass, a basal metabolism key 27 for displaying basal metabolism, and a visceral fat key 28 for displaying visceral fat area.

The part-of-body selection key sub-group 20 is provided for selecting a part of the body to be displayed, and is consisting of a whole body key 29, a right arm key 30, a left arm key 31, a right foot key 32, a left foot key 33, and a trunk key 34.

Fig. 3 is an electrical block diagram of the body composition meter 1 in Fig. 1. In particular, eight (8) electrodes 4a, 4b, 5a, 5b, 11a, 11b, 13a, 13b for right and left hands and feet are connected to an electrode switching unit 35. The electrode switching unit 35 is connected to a controller unit 38 via a current supplying unit 36 and a voltage measurement unit 37. The controller unit 38 is provided for controlling the entire body composition meter 1, and has a microcomputer mounted inside and a memory unit 39 mounted outside for storing various types of data. The controller unit 38 is connected to a power supply 40. The display unit 7 is connected to the controller unit 38 via an input / output control unit 41. Similarly, the operation unit 6, the personal keys 8a-8d, and the body weight / OFF key 9 are connected to the controller unit 38 via an input / output control unit 41. Furthermore, the body weight detection unit 2 within the body composition meter 1 is connected to the controller unit 38.

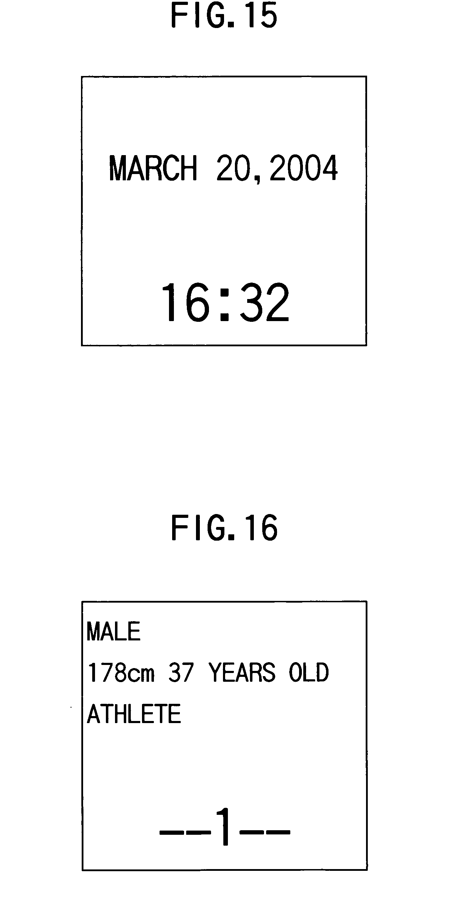

Now, operation of the body composition meter 1 will be described in more detail with reference to the configuration diagrams of Figs. 1 to 3, flow charts of Figs. 4 to 14, and display screen views of Figs. 15 to 27.

At first, a rear cover of the body composition meter 1 is opened and a dry battery is inserted into the power supply unit in Fig. 3. Then, the controller unit 38 acts to set a calendar or current date and time according to the flow charts of Figs. 4A, 4B and 4C. At step S1 the microcomputer memory, counter, etc. in the controller unit 38 is reset and the calendar is set at "0:00, January 1, 2004" as the initial calendar data. Then, at step S2, any one key of the operation key group 17 consisting of the SET key 14, DOWN key 15 and UP key 16 is depressed, and then, the characters representing such calendar data are continuously displayed, except for the calendar year "2004" which is flashed on the display unit 7, at step S3. If the value for calendar year corresponds to the current calendar year then the SET key 14 is depressed, at step S4, to store the value for calendar year in the memory unit 39.

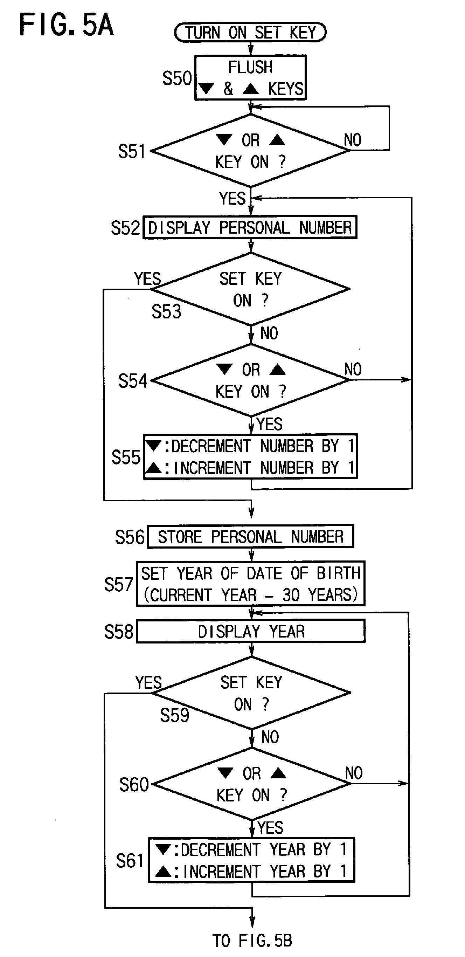

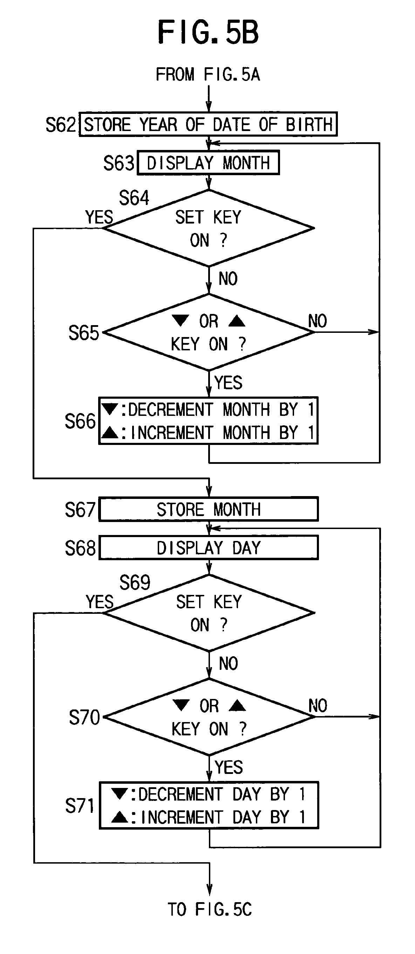

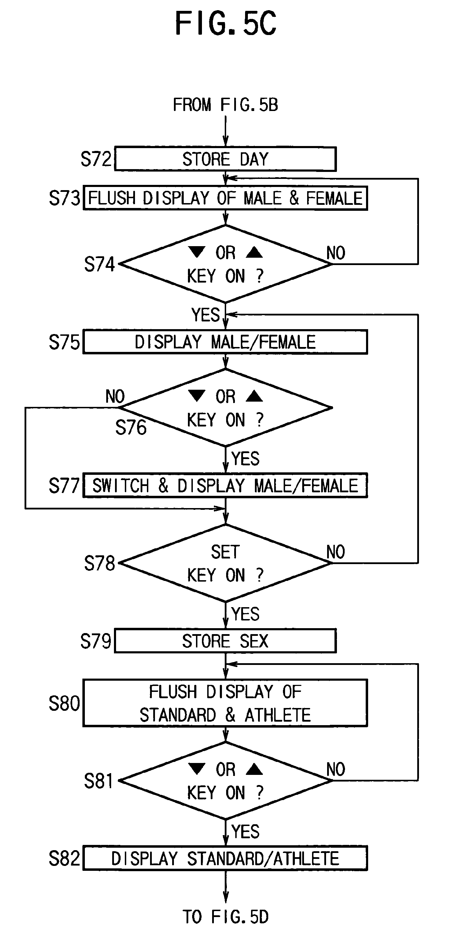

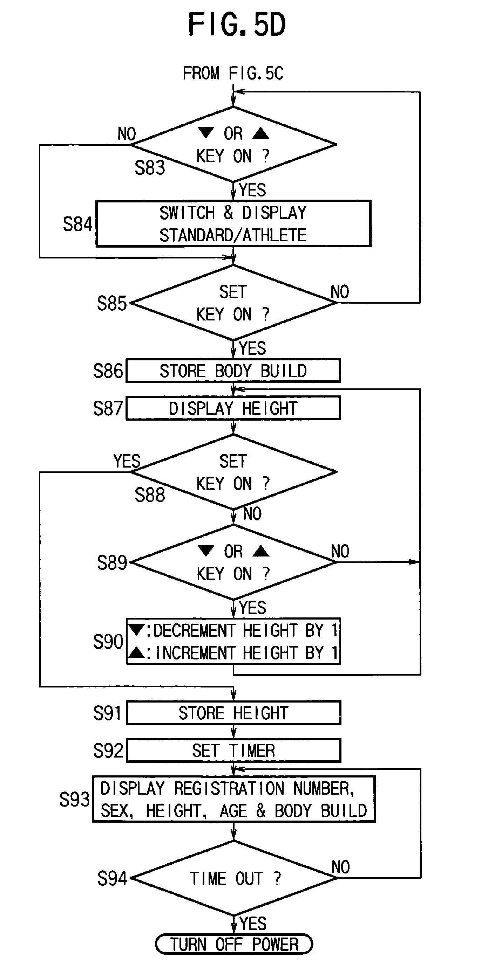

However, if it is desired to change the current calendar year, the SET key 14 is not depressed, but the UP key 16 or Down key 15 is depressed at step S5 to increase or decrease the value for calendar year by one at a time, at step S6, and thereafter, the value for calendar year is displayed at step S3. It is noted that if the value would be increased or decreased beyond the upper or lower limit set for the apparatus then it is looped from the lower limit to upper limit or vice versa to change the numeral value displayed. Such process of changing the value by looping is commonly applied to the case of UP or DOWN operation of display of other data such as month, time, height, age, etc., as described hereafter with reference to Figs. 4A to 4C and 5A to 5D.

After the value for current calendar year is stored at step S7 then the month "January" is flashed on the display unit 7 at step S8. Setting for the current month is performed at steps S9 to S12, in the same manner as setting for the current calendar year, as described above with reference to steps S4 to S7. Similarly, setting for the current day is performed at steps S13 to S17, setting for the current hour at steps S18 to S22, and setting for current minute at steps S23 to S27, respectively.

After settings for year, month, day, hour and minute have been done, a timer for approx. 3 sec. is set at step S28 to display the current date and time, i.e. current calendar year, month, day, hour and minute on the display unit 7 at step S29, as shown in Fig. 15. When the timer that is set at step S28 is expired, as found at step S30, then the power is turned OFF. It is noted that the microcomputer in the controller unit 38 is kept operated even when the power is turned OFF unless the battery is removed. Accordingly, the current date and time is updated under the control of the controller unit 38. In addition, when the operation key group 17, the personal keys 8a-8b, the body weight / OFF key 9, or any other key is depressed, the body composition meter 1 is started under the control of the controller unit 38.

Next, registration of the personal data of a person under test with the body composition meter 1 will be described with reference to the flow charts of Figs. 5A to 5D. At first, the SET key 14 is turned ON to display the marks "▼" and "▲" for the DOWN key 15 and the UP key 16 which are flashed on the display unit 7, at step S50. Then, the DOWN key 15 or the UP key 16 is turned ON (at step S51) to display "1" of the personal numbers "1"to "4" which correspond to the personal keys 8a-8d, at step S52. Then, the DOWN key 15 is turned ON to display the personal number "4" or the UP key 16 is turned ON to display the personal number "2". If it is desired to register the data with the personal number displayed then the SET key 14 is turned ON at step S53 to proceed to step S56 where that personal number is stored in the memory unit 39.

However, if it is desired to change the personal number displayed then the DOWN key 15 or the UP key 16 is turned ON at step S54 to decrease or increase the personal number by one at step S55. Then, the personal number is displayed on the display unit 7 at step S52. This operation is repeated until the desired personal number is selected.

After the personal number is registered at step S56 then the calendar year of the date of birth of the person under test is set at step S57. More particularly, the current calendar year as set in Fig. 4 minus 30 years is calculated to display the resultant calendar year as the initial value at step S58. Setting of the calendar year of the date of birth is performed at steps S58 to S62, which are similar to the steps S3 to S7 in Fig. 4A. Therefore, further description for those steps is omitted here.

After setting of the calendar year of the date of birth is completed at step S62 then the month and the day of the date of birth are set at steps S63 to S67 and steps S68 to S72, respectively, which are similar to the steps S8 to S12 and steps S13 to S17 in Fig. 4B. Therefore, further description for those steps is omitted here.

After setting of the date of birth is completed then the characters representing male and female are flashed on the display unit 7 to determine whether the person under test is male or female at step S73. Then, the DOWN key 15 or the UP key 16 is turned ON (step S74) to display the characters representing male or female at step S75. In particular, the DOWN key 15 is turned ON to display "Male" or the UP key 16 is turned ON to display "Female" on the display unit. If it is desired to register with the sex displayed then the SET key 14 is turned ON at step S78 without further depressing of the DOWN key 15 or the UP key 16 (at step S76) to proceed to step S79 where the sex displayed is stored in the memory unit 39.

However, if it is desired to change the sex displayed then the DOWN key 15 or the UP key 16 is turned ON at step S76 to switch and display "Male" or "Female" at step S77. It is noted that if the steps S76 and S77 are repeated without depressing the SET key 14 then the sex can selectively be displayed.

After setting of the sex is completed then the routine proceeds to steps S80 to S86 where body build of the person under test is determined as to whether he/she is standard type or athlete type. Those steps are similar to the steps S73 to S79 except that the setting of sex, i.e. male or female is replaced with that of standard or athlete type. As the result, further description thereof is omitted here.

Next, the routine proceeds to step S87 where setting of height of the person under test is performed. An initial value of 170 cm is displayed on the display unit 7. If this value is OK then the SET key 14 is turned ON at step S88 to proceed to step S91 where it is stored in the memory unit 39.

However, if it is desired to change the height value displayed then the DOWN key 15 or the UP key 16 is turned ON (at step S89) to decrease or increase the height value by 1 cm at step S90 and to display it on the display unit 7 at step S87.

After completion of setting of the height then the timer for approx. 3 sec. is set at step S92 to display the personal number, sex, height, current age calculated under the controller unit 38 based on the current date and the date of birth, and body build of the person under test, at step S93, as shown in Fig. 16. This personal data is kept displayed until the timer is expired at step S94, upon which the power is turned OFF.

After setting of the calendar and registration of the personal data have been done, as described with reference to Figs. 4A to 4C and 5A to 5D, the body composition meter 1 is ready for measurement.

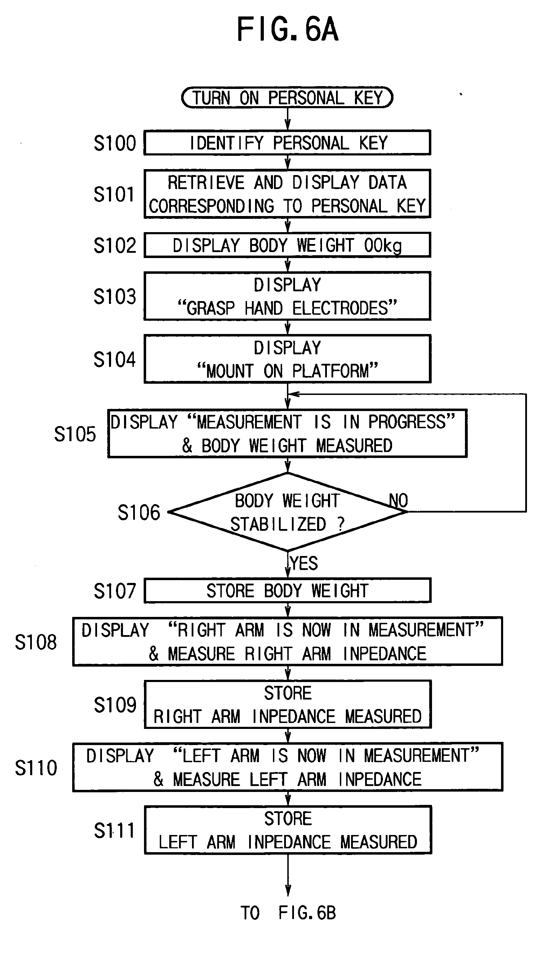

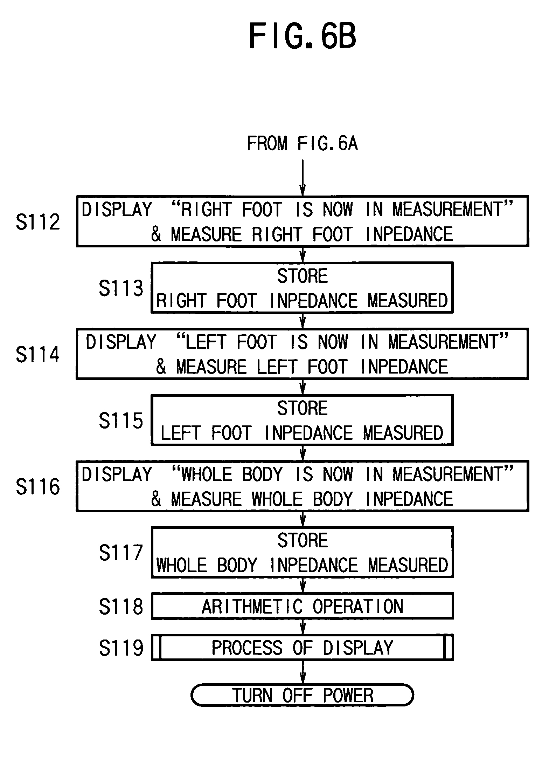

Now, measurement will be described with reference to the flow charts of Figs. 6A and 6B. At first, any one of the personal keys 8a-8d on the body composition meter 1 is depressed by e.g. a tiptoe of the person under test to start the measurement under the control of the controller circuit 38 according to the flow charts of Figs. 6A and 6B. At step S100, which one of the personal keys 8a-8d is depressed is determined, and the personal data of the personal number corresponding to that key is retrieved from the memory unit 39 to display it on the display unit 7 at step S101, as shown in Fig. 16. At this time, if there is no personal data corresponding to the key depressed stored in the memory unit 39 (that is to say, no registration of the personal data has been done) then some message is displayed on the display unit 7 for informing that there is no registration of personal data and the power is turned OFF after approx. 3 sec. later.

After the personal data displayed on the display unit 7 for a fixed period at step S101 then display "0.0 kg" is appeared on the display unit 7 at step S102, as shown in Fig. 2, and some message "Grasp hand electrodes" is appeared above the display "0.0 kg" at step S103. After the time period of several seconds, a message "Mount on the platform" is displayed for measuring the body weight at step S104.

When the person under test mounts on the platform the body weight detection unit 2 detects the body weight of the person so that a message "Measurement is in progress" is displayed on the display unit 7, together with the body weight measured, at step S105. Because the body weight measured tends to be fluctuated even with slight movement of the person under test it is necessary to determine whether the body weight measured is stabilized within the predetermined range of e.g. 0.05 kg, at step S 106. If so, the body weight measured is stored in the memory unit 39 at step S107.

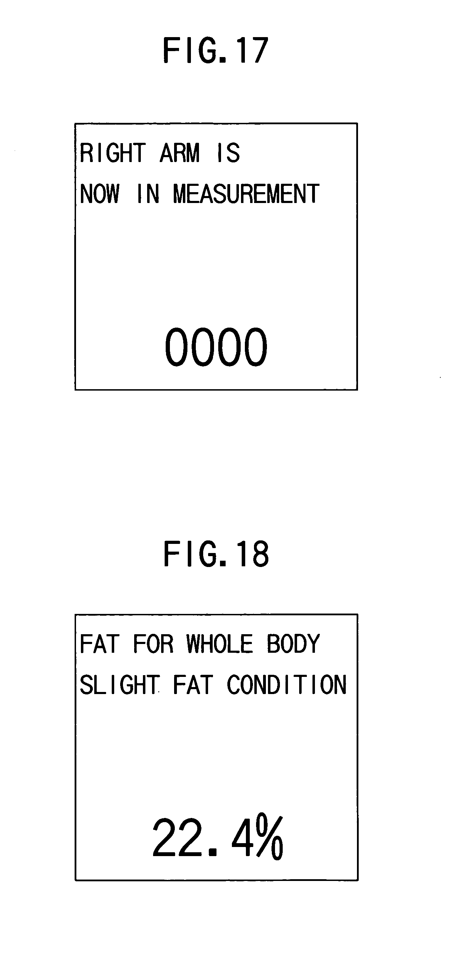

After completion of measurement of body weight a message "Right arm is now in measurement" is displayed on the display unit 7, together with display "0000" that shows the progress of measurement, at step S108, as shown in Fig. 17. In particular, the display "0000" is successively extinguished, starting from the leftmost "0", with the progress of measurement, and ultimately, extinction of all "0"s means that measurement is completed. Measurement of the right arm is performed in such manner that the controller unit 38 controls the electrode switching unit 35 so that the current supplying unit 36 supplies the constant current at the predetermined frequency to the right hand electrode 11a and the right foot electrode 4a and the voltage measurement unit 37 measures the voltage between the right hand electrode 11b and the left hand electrode 13b. The controller unit 38 calculates bioelectrical impedance of the right hand, based on the constant current and the voltage measured, which bioelectrical impedance is then stored in the memory unit 39 (at step S109). After measurement of the right hand, at step S110, a message "Left arm is now in measurement" is displayed in the same manner as in Fig. 17. Then, similar to the case of the right hand, the current supplying unit 36 supplies the current to the left hand electrode 13a and the left foot electrode 5a and the voltage measurement unit 37 measures the voltage between the left hand electrode 13b and the right hand electrode 11b. The controller unit 38 calculates bioelectrical impedance of the left hand, which is then stored in the memory unit 39 (at step S111).

After measurement of the left hand, measurement of the right foot, the left foot and the whole body is performed at steps S112 to S117. However, the electrode switching operation and the bioelectrical impedance measurement are already known from the commercial body composition meters that have been put into market, and therefore, further description thereof is omitted here.

After measurement of impedance for each of parts, including that for a whole body, has been done, then, at step S118, the weight, body fat rate, muscle mass, bone mass, and body water mass for a whole body and for each of parts, as well as the basal metabolism for a whole body and the visceral fat area for a trunk portion are calculated, based on the personal data entered and registered as described in Figs. 5A to 5D, the body weight measured, the bioelectrical impedance measured for each of parts, etc. Method of calculation is already known from the body composition meters that have been put into market, and therefore, further description thereof is omitted here. The result of calculation is stored in the memory unit 39, together with the date and time at which the measurement has been done for each of the personal numbers.

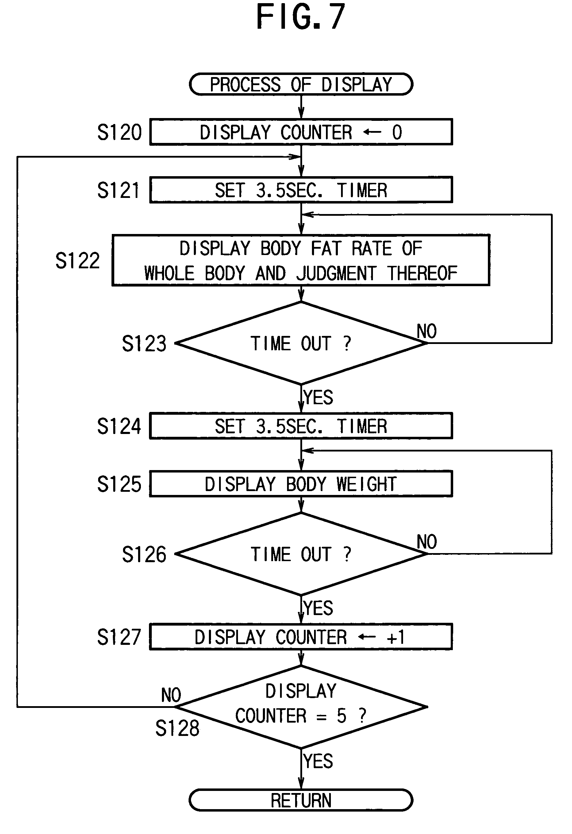

At step S 119 the body fat rate of a whole body selected among the various measurement data obtained and the result of judgment thereof as well as the body weight of a whole body are displayed on the display unit 7 for a fixed period, and thereafter, the power is turned OFF. This display will be described later in more detail with reference to Fig. 7.

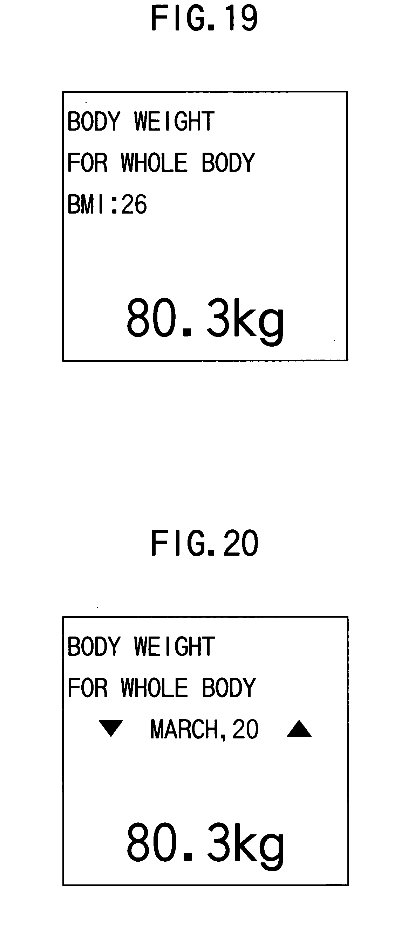

Referring to the sub-routine in Fig. 7, at step S120, a display counter for indicating how many times display is made is cleared, and at step S121, a 3.5 sec. timer is set for setting the time period during which display is made at step S122. At step S122, "Whole Body" which means that the part of the body displayed is a whole body, "Fat" which means the fat rate, a numerical value of body fat rate of a whole body which is calculated at step S 118 in Fig. 6B, and the result of judgment, for example, "Slightly Fat Condition", are displayed on the display unit 7, as shown in Fig. 18. Then, at step S 123, it is determined whether such display has been kept for time period of 3.5 sec., that is to say, whether the 3.5 sec. timer is expired. If the timer is expired, the sub-routine proceeds to step S124 where the 3.5 sec. timer is set once again. Then, at step S125, the body weight of a whole body and "BMI" are displayed on the display unit 7, as shown in Fig. 19. Then, at step S126, it is determined whether such display has been kept for time period of 3.5 sec., that is to say, whether the 3.5 sec. timer is expired. If the timer is expired, the sub-routine proceeds to step S127 where the display counter is incremented by 1. Then, at step 128, the count of the display counter indicating how many times a combination of two displays as above is repeated is checked to determine whether the count reaches the predetermined number of times (in this example, five (5) times). If the count does not reach it the sub-routine returns to step S121 to repeat the display operation. If the count reaches the predetermined number of times, however, the sub-routine restores step S 119 in Fig. 6B to turn the power OFF.

The sub-routine of display, as described above, is standard in this embodiment of the body composition meter 1. Additionally, if any one key of the display selection key group 18 is turned ON during the sub-routine of display in Fig. 7 then the body composition meter 1 can be operated so that the data corresponding to that key is retrieved from the memory unit 39 and is displayed on the display unit 7. The display selection key group 18 includes a display item selection key sub-group 19 for selecting a display item for the body compositions such as body fat rate, bone mass, etc., a part-of-body selection key sub-group 20 for selecting a part of the body such as a whole body, right arm, etc., and a graph selection key 21 for selecting a display among numerical data display, daily graphic display, weekly graphic display and monthly graphic display.

Three control counters are provided each for each of those three selection key sub-groups so that the proper numerical value is assigned to each of the counters depending on the key that is turned ON and on the number of times that the key is turned ON. The content of display on the display unit 7 is controlled according to the numerical values of those three counters.

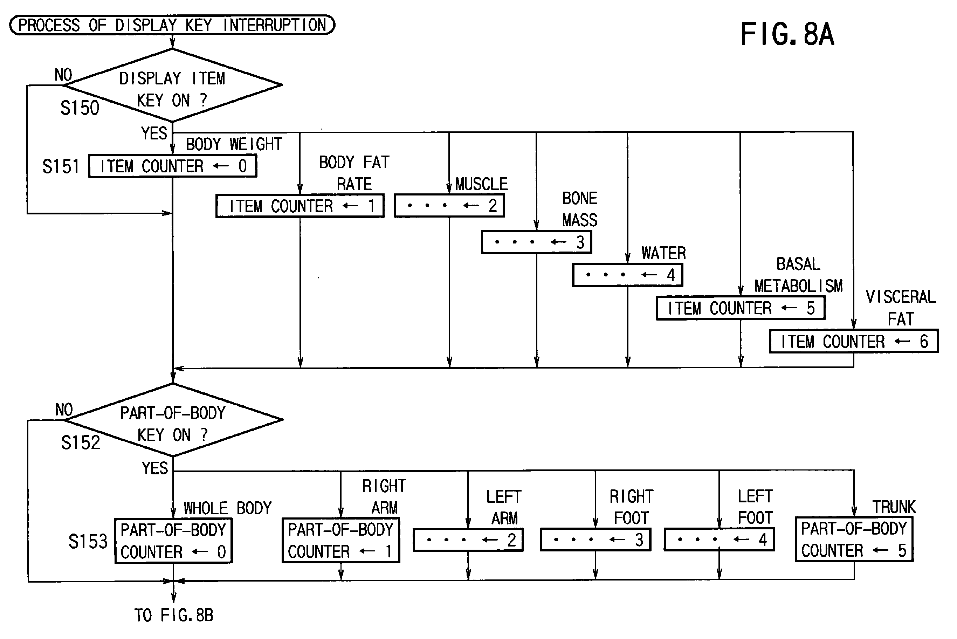

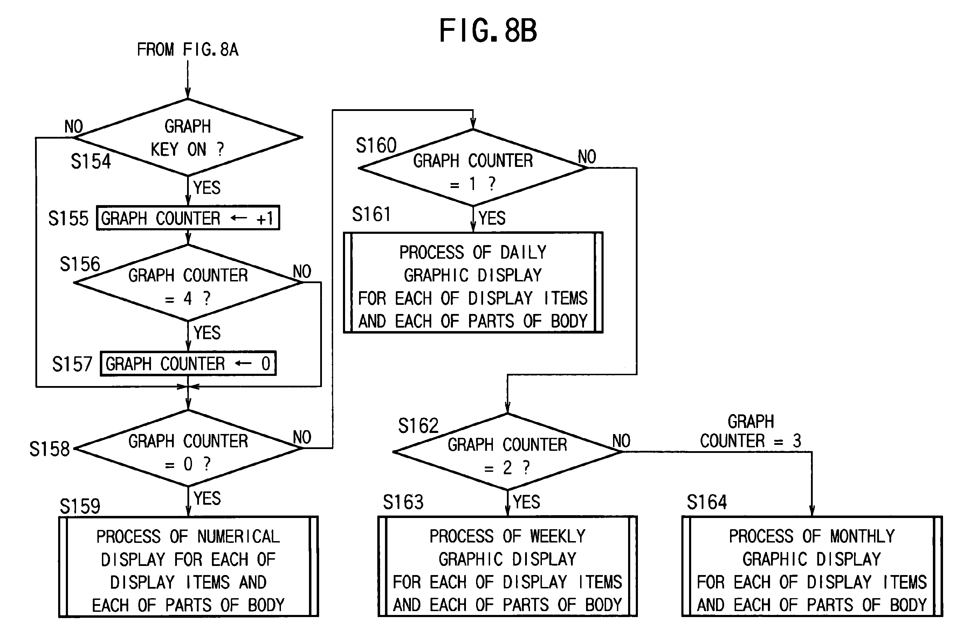

The control operation is carried out according to the flow charts in Fig. 8A and 8B. At first, any one key of the display selection key group 18 is turned ON to start an interruption to the microcomputer in the controller unit 38 so that the process is transferred from the sub-routine of display in Fig. 7 to the routine in Figs. 8A and 8B.

If the key that is turned ON at step S 150 is any one of the display item selection key sub-group 19 then at step S151 the numerical value assigned to an item counter is set depending on which key of the display item selection key sub-group 19 is turned ON. For example, if the body weight key 22 is turned ON the item counter is set at "0", but if the body fat key 23 is turned ON the item counter is set at "1".

On the other hand, if the key that is turned ON is any one of the part-of-body selection key sub-group 20 then at step S152 the numerical value assigned to a part-of-body counter is set depending on which key of the part-of-body selection key sub-group 20 is turned ON. For example, if the whole body key 29 is turned ON the part-of-body counter is set at "0", but if the right arm key 30 is turned ON the part-of-body counter is set at "1".

Furthermore, if the key that is turned ON is the graph key 21 then the answer of step S 154 is "Yes" and a graph counter is incremented by 1 at step S155. Then, at step S156, it is determined whether the graph counter reaches "4". If the graph counter reaches "4" it is assumed that the graph key 21 is turned ON while the monthly graph is displayed. Accordingly, the graph counter is reset to "0" (at step S157), which return the display to numerical value mode. However, if the graph counter does not reach "4" at step S 156 then the routine proceeds to step S 158 where it is determined whether the graph counter is "0".

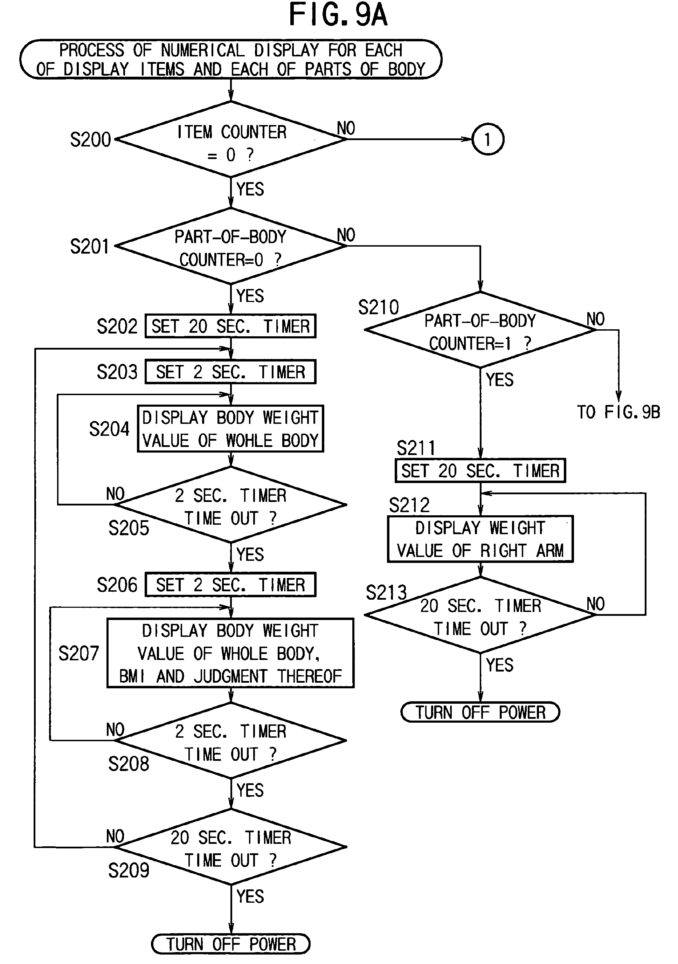

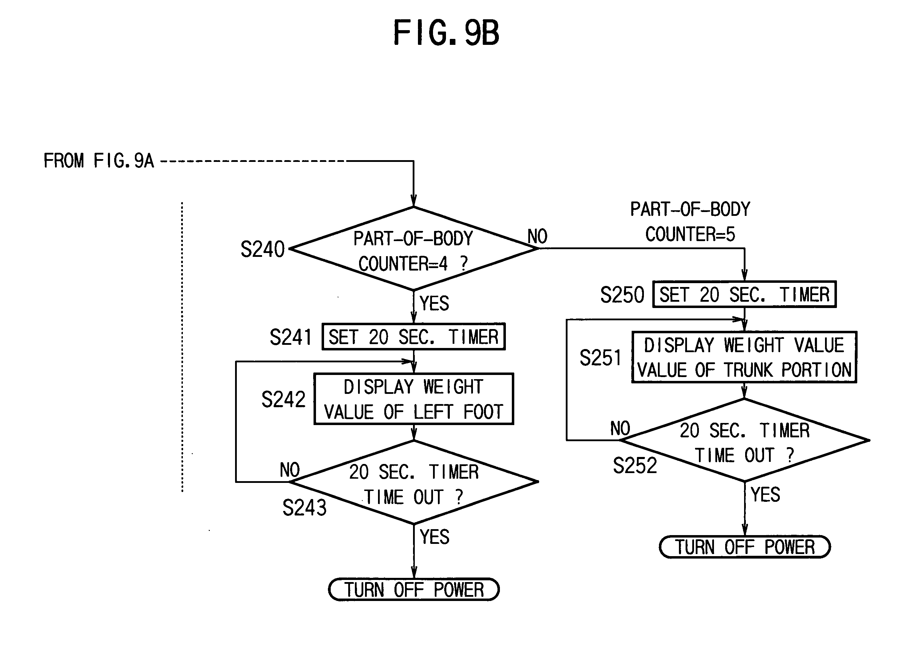

If the graph counter is "0" at step S158 then the routine proceeds to step S 159 where process of numerical display for each of display items and for each of parts of the body is performed. This type of display process is conducted according to the sub-routines in Figs. 9 to 11. If any one key of the display selection key group 18 would be turned ON again during process of these sub-routines then any interruption is caused to the microcomputer so that the routine returns to the interruption process according to the flow charts in Figs. 8A and 8B.

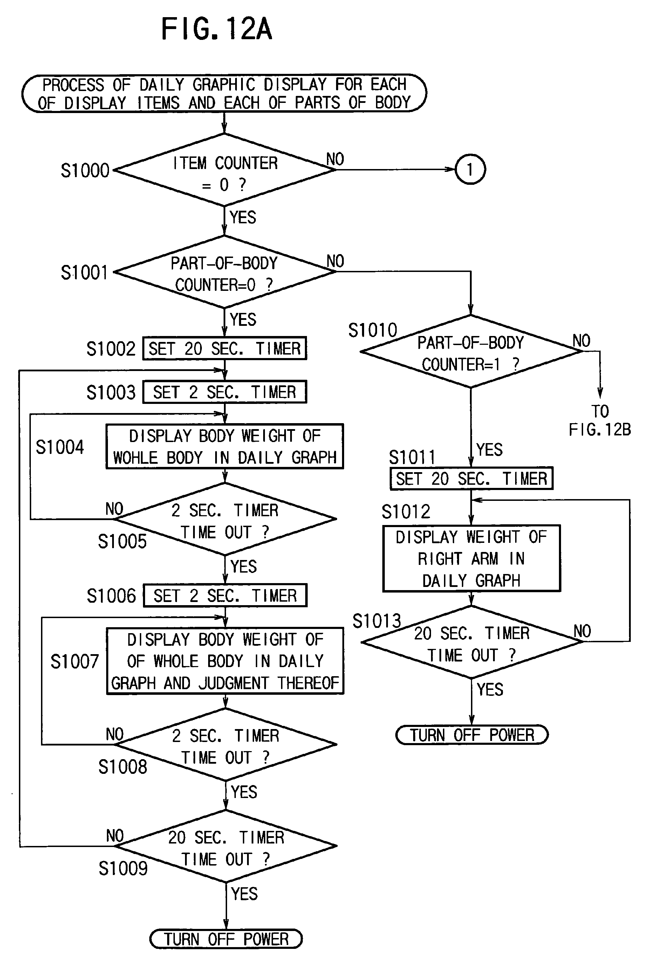

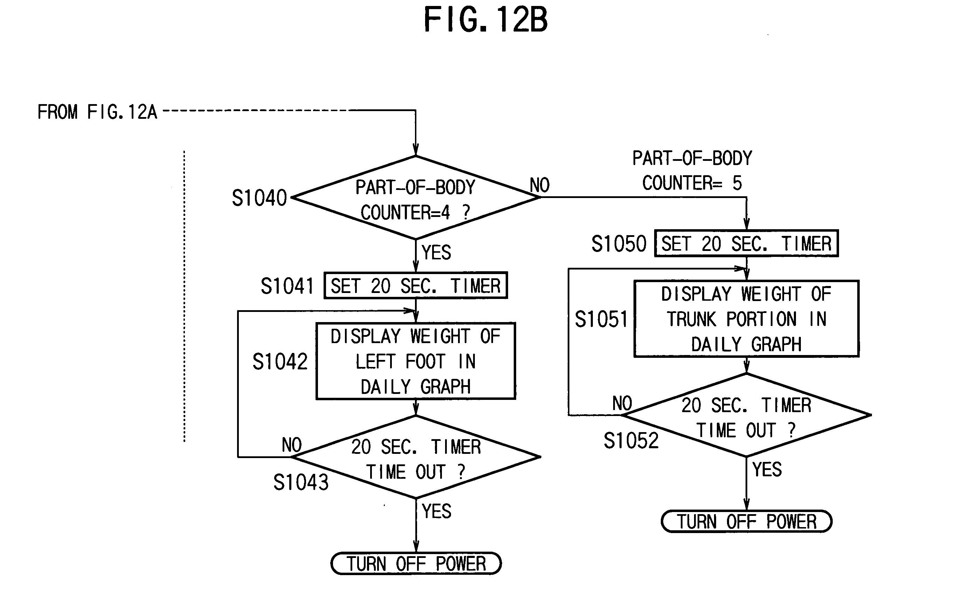

If the answer of step S158 is "No" the routine proceeds to step S160 where it is determined whether the graph counter is "1". If the graph counter is "1" then the routine proceeds to step S161 where process of daily graphic display for each of display items and for each of parts of the body is performed. This type of display process is conducted according to the sub-routines in Figs. 12 to 14. If any one key of the display selection key group 18 would be turned ON again during process of these sub-routines then any interruption is caused to the microcomputer so that the routine returns to the interruption process according to the flow charts in Fig. 8A and 8B.

If the answer of step S160 is "No" the routine proceeds to step S162 where it is determined whether the graph counter is "2". If the graph counter is "2" then the routine proceeds to step S163 where process of weekly graphic display for each of display items and for each of parts of the body is performed. If the answer of step S162 is "No" then the graphic counter is "3" so that the routine proceeds to step S164 where process of monthly graphic display for each of display items and for each of parts of the body is performed. It is noted, here, that the display process at steps S163 and S164 is same as that of step S161 except that daily unit on abscissa of the graph is replaced with weekly or monthly unit. Accordingly, further description of the sub-routines for weekly and monthly graphic displays is omitted, here. If any one key of the display selection key group 18 would be turned ON again during process of these sub-routines then any interruption is caused to the microcomputer so that the routine returns to the interruption process according to the flow charts in Figs. 8A and 8B.

Referring to Figs. 9 to 11, the process of numerical display for each of display items and each of parts of the body at step S159 in Fig. 8B will be described in more detail.

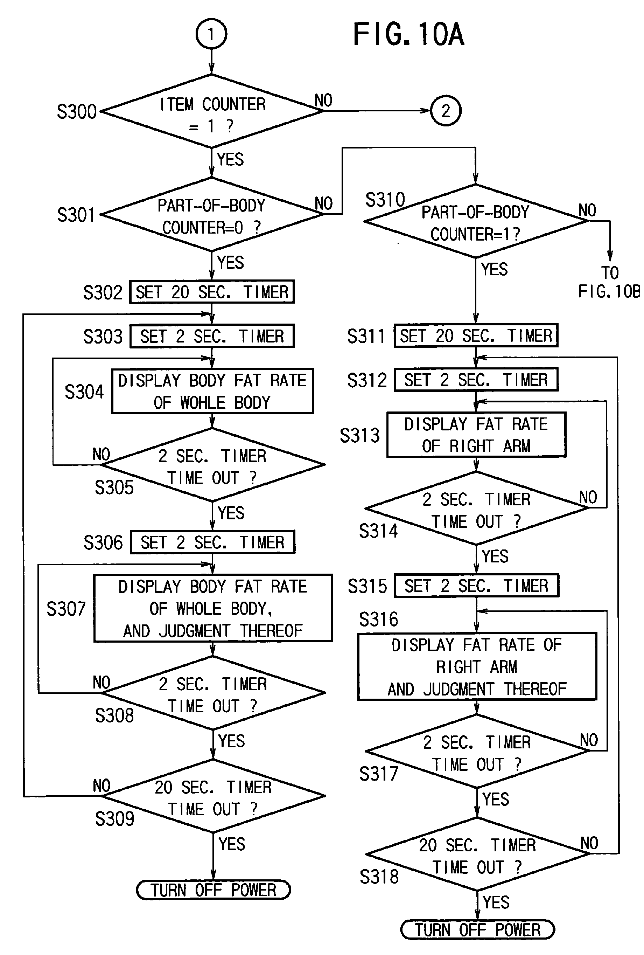

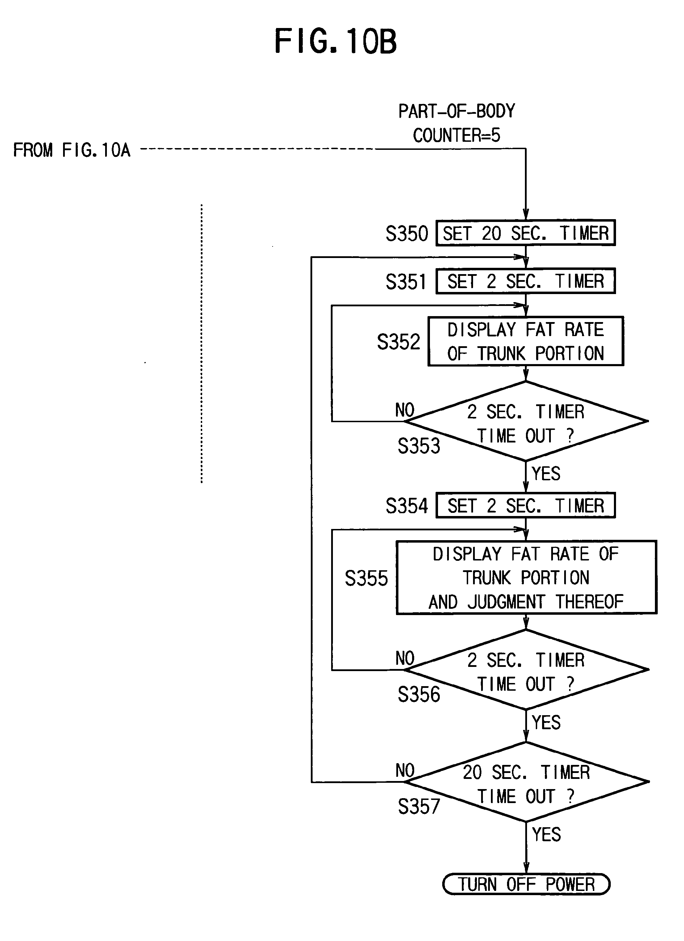

At step S200 it is determined whether the item counter that is set as shown in Fig. 8 is "0" or not. If it is "0" the routine proceeds to step S201 to display the body weight. However, if it is not "0" at step S200 the answer thereof is "No" so that the routine proceeds to step S300 in Fig. 10A.

At step S201 it is determined whether the part-of-body counter that is set as shown in Fig. 8 is "0" or not. If it is "0" which means that whole body is selected for the part-of-body, then the routine proceeds to step S202 where a 20 sec. timer is set for setting the period of display until the power is turned OFF. Then, at step S203, a 2 sec. timer is set during which period the body weight of whole body is displayed at step S204. In particular, at step S204, the body weight of whole body that is measured at steps S 105 to S107 in Fig. 6A and is stored in the memory unit 39 is displayed on the display unit 7, together with the date on which the measurement is made, as shown in Fig. 20.

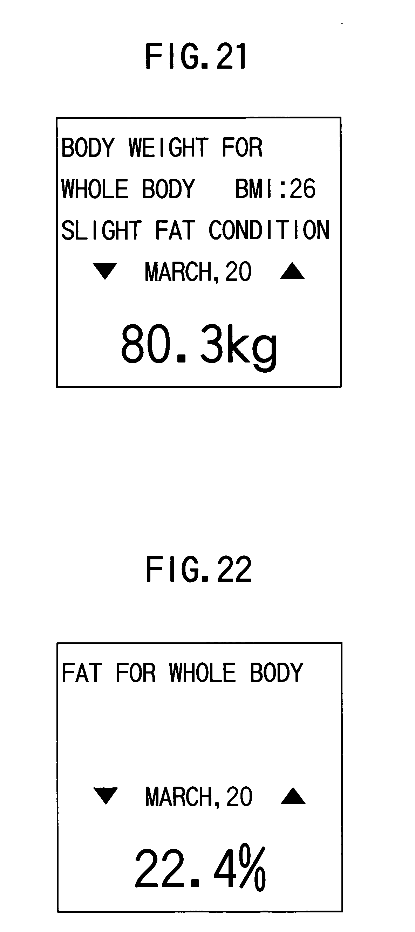

Then, at step S205, it is determined whether the 2 sec. timer is expired or not. If the timer is not expired the routine returns to step S204 where the body weight of whole body is displayed. However, if the timer is expired the routine proceeds to step S206 where the 2 sec. timer is set again. Then, at step S207, the date on which the measurement is performed, the characters representing the body weight of whole body and the numerical value of whole body weight, the numerical value of body mass index BMI, and the judgment for adiposity based on the BMI, e.g. "Slightly Fat Condition", are displayed, as shown in Fig. 21. Then, at step S208, it is determined whether the timer is expired or not. If so, the routine proceeds to step S209. At this step S209 it is determined whether the 20 sec. timer that has been set at step S202 is expired or not. If not, the routine returns to step S203 and the two types of display as described above are repeated. If they are repeated five times then the total time that has passed amounts to 20 sec., upon which it is determined that the timer is expired at step S209, and as the result, the power is turned OFF.

In steps S202 to S209 the latest measurement value and the date on which the measurement is made are displayed. However, if the DOWN key 15 in Fig. 2 is turned ON while they are displayed, then the date of the previous day and the measurement value obtained on that day are displayed. Then, if the DOWN key 15 is turned ON once again then the date of two days before and the measurement value obtained on that day are displayed. On the other hand, if the UP key 16 is turned ON two times then the latest measurement value and the date of measurement are again displayed. In such manner, operation of the DOWN key 15 or the UP key 16 in the course of display can make change the date of measurement for which the measurement value is to be displayed. But, if there is no measurement data available on the day that is specified in such way, a message "No Data Available" is displayed on the display unit 7 because of no measurement value stored.

If the part-of-body counter is "1", instead of "0", at step S201 then the answer of step S210 is "Yes". Then, the routine proceeds to step S211 where the 20 sec. timer is set. Then, at step S212, the weight of right arm that is calculated based on the data such as height, body weight, and bioelectrical impedance for each of parts of the person under test at step S118 in Fig. 6B and stored in the memory unit 39 is retrieved and displayed. Then, expiration of the timer is waited at step S213, and the power is turned OFF. For weight of right arm its numerical value is simply displayed in this example because of no judgment criterion provided for classifying it into standard, adiposity, etc. If in future some judgment criterion becomes available then two displays, i.e. numerical value of and judgment for weight of right arm can alternately be presented, as with the body weight of whole body at steps S203 to S208.

If the DOWN key 15 in Fig. 2 is turned ON while the weight of right arm is displayed, then the measurement value obtained on the previous day is displayed. Furthermore, if the UP key 16 is turned ON then the latest measurement value is again displayed. In such manner, operation of the DOWN key 15 or the UP key 16 in the course of display can make change the date of measurement for which the measurement value is to be displayed. But, if there is no measurement data available on the day that is specified in such way, a message "No Data Available" is displayed on the display unit 7 because of no measurement value stored.

If the answer of step S210 is "No" then the part-of-body counter may indicate any one of 2 to 5. In Fig. 9 branch paths for the case of the part-of-body counter = 2, 3, 4 and 5 are generally same, except for no decision block provided for the part-of-body counter = 5. Therefore, branch paths for the part-of-body counter = "2" and "3" and the corresponding descriptions are omitted, here.

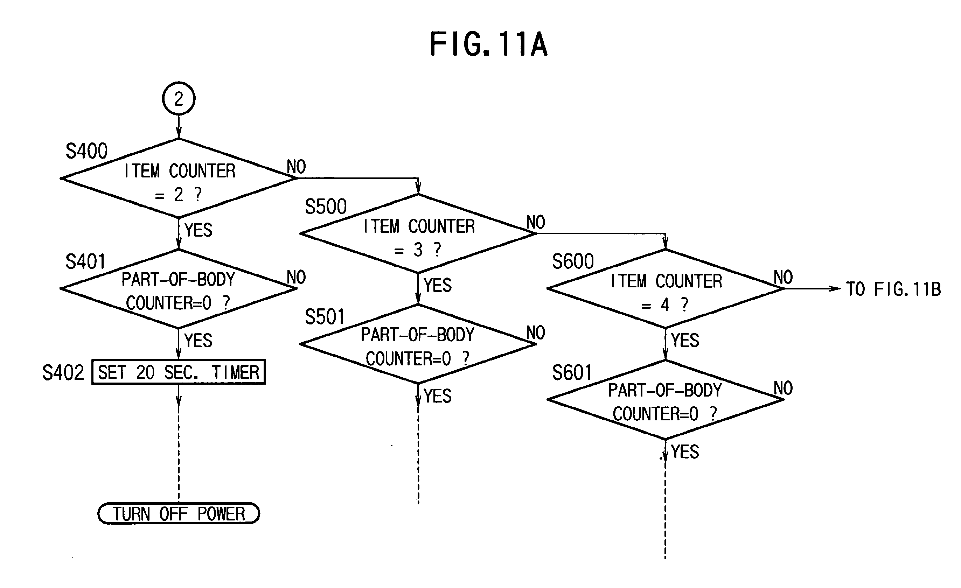

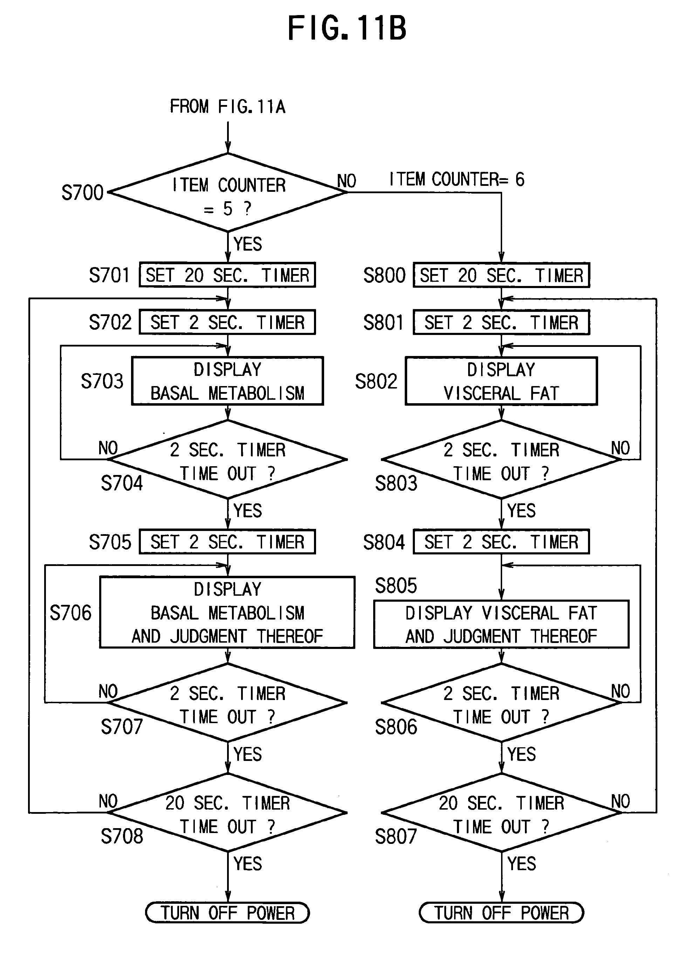

If the answer of step S200 in Fig. 9A is "No" the routine proceeds to step S300 in Fig. 10A. At step S300 it is determined whether the item counter that is set as shown in Fig. 8 is "1" or not. If so, the routine proceeds to step S301 to display the body fat rate. However, if not, the answer is "No" so that the routine proceeds to step S400 in Fig. 11A.

At step S301 it is determined whether the part-of-body counter is "0" or not. If it is "0" which means that whole body is selected for the part of body then the routine proceeds to step S302 where the 20 sec. timer is set for setting the time period during which display is made until the power is turned OFF. Then, at step S303, the 2 sec. timer is set during which period the body fat rate of whole body is displayed at step S304. In particular, at step S304, the body fat rate of whole body that is measured at steps S 105 to S 107 in Fig. 6A and is stored in the memory unit 39 is displayed on the display unit 7, as shown in Fig. 22.

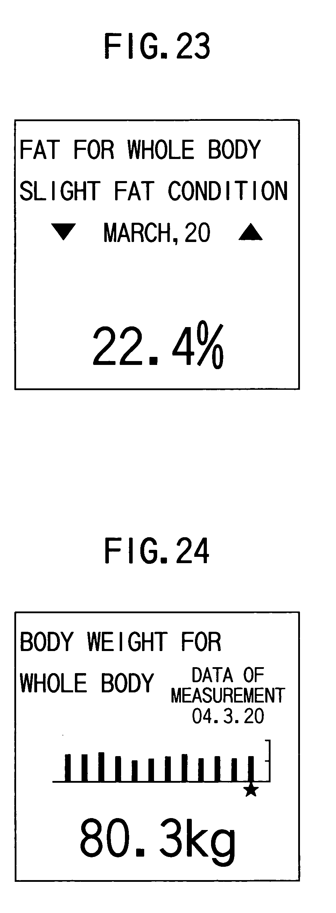

Then, at step S305, it is determined whether the 2 sec. timer is expired or not. If the timer is not expired the routine returns to step S304 where the body fat rate of whole body is displayed. However, if the timer is expired the routine proceeds to step S306 where the 2 sec. timer is set again. Then, at step S307, characters representing the body fate rate of whole body and the numerical value thereof, and the judgment for adiposity, e.g. "Slightly Fat Condition", are displayed, as shown in Fig. 23. Then, at step S308, it is determined whether the timer is expired or not. If so, the routine proceeds to step S309. At this step S309 it is determined whether the 20 sec. timer that has been set at step S302 is expired or not. If not, the routine returns to step S303 and the two types of display as described above are repeated. If they are repeated five times then the total time that has passed amounts to 20 sec., upon which it is determined that the timer is expired at step S309, and as the result, the power is turned OFF.

In steps S302 to S309 the latest measurement value is displayed. However, if the DOWN key 15 in Fig. 2 is turned ON while it is displayed, then the measurement value obtained on the previous day is displayed. Furthermore, if the UP key 16 is turned ON then the latest measurement value is again displayed. In such manner, operation of the DOWN key 15 or the UP key 16 in the course of display can make change the date of measurement for which the measurement value is to be displayed. But, if there is no measurement data available on the day that is specified in such way, a message "No Data Available" is displayed on the display unit 7 because of no measurement value stored.

If the part-of-body counter is "1", instead of "0", at step S301 then the answer of step S310 is "Yes". Then, the routine proceeds to step S311 where the 20 sec. timer is set. Then, at step S312, the 2 sec. timer is set during which period the fat rate of right arm is displayed at step S313. In particular, at step S313, the fat rate of right arm that is calculated at step S 119 in Fig. 6B and stored in the memory unit 39 is retrieved and displayed. Then, expiration of the timer is waited at step S314, and then, the routine proceeds to step S315 where the 2 sec. timer is set during which period display is made at step S316. At this step S316 the fat rate of right arm and the result of judgment thereof stored in the memory unit 39 are displayed. Expiration of the timer is waited at step S317, and then, the routine proceeds to step S318. At this step S318, it is determined whether the 20 sec. timer that has been set at step S311 is expired or not. If not, the routine returns to step S312 and the two types of display as described above are repeated. If they are repeated five times then the total time that has passed amounts to 20 sec., upon which it is determined that the timer is expired at step S318, and as the result, the power is turned OFF.

In steps S311 to S318 the latest measurement value is displayed. However, if the DOWN key 15 in Fig. 2 is turned ON while it is displayed, then the measurement value obtained on the previous day is displayed. Furthermore, if the UP key 16 is turned ON then the latest measurement value is again displayed. In such manner, operation of the DOWN key 15 or the UP key 16 in the course of display can make change the date of measurement for which the measurement value is to be displayed. But, if there is no measurement data available on the day that is specified in such way, a message "No Data Available" is displayed on the display unit 7 because of no measurement value stored.

If the answer of step S310 is "No" then the part-of-body counter may indicate any one of 2 to 5. In Fig. 10 branch paths for the case of the part-of-body counter = 2, 3, 4 and 5 are generally same, except for display items. Therefore, branch paths for the part-of-body counter and the corresponding descriptions are partially omitted, here.

If the item counter is not "1" at step S300 in Fig. 10A the answer thereof is "No" so that the routine proceeds to step S400 in Fig. 11A. If the item counter is "2" at step S400 then the routine proceeds to step S401 and the subsequent steps because of display of muscle mass selected in Fig. 8. The flow chart representing such routine and the corresponding description are omitted, here, because the display item, i.e. body fat rate of the body fat rate display process in Fig. 10 is only replaced with the muscle mass.

Furthermore, if it is determined that the item counter is "3" in step S500 then the routine proceeds to step S501. In this case, the display item is bone mass. In addition, if the item counter is "4" in step S600 then the routine proceeds to step S601. In this case, the display item is body water mass. Moreover, if the item counter is "5" in step S700 then the routine proceeds to step S701. In this case, the display item is basal metabolism. Furthermore, if the item counter is "6" in step S700 then the routine proceeds to step S800. In this case, the display item is visceral fat area. The flow chart representing such routine and the corresponding description are omitted, here, because the display item in the case of process in Fig. 10 is only replaced with the relevant one.

It is noted, here, that in steps S700 and S800 and their subsequent steps displays of basal metabolism and visceral fat area are handled. However, measurement and judgment method for basal metabolism for each of parts of the body has not been developed in the art, and therefore, only basal metabolism for whole body and the judgment thereof are handled, here. In addition, the visceral fat area has the meaning only for the trunk portion of the body, and therefore, there is no display of visceral fat area handled, here, for each of parts of the body.

In the process of numerical display for each of display items and each of parts of body, as shown in Figs. 9 to 11, if it is desired to provide an additional display for desired display item or desired part of body while one display is provided, then any relevant key selected among the display item selection key sub-group 19 and the part-of-body selection key sub-group 20 in Fig. 2 may be turned ON to cause a display key interruption process as shown in Fig. 8 so that the process of display in Figs 9 to 11 is executed to provide the desired display. That is to say, the person under test may simply turn the desired key ON to provide the desired display without any restriction to some operation procedure.

Now, the process of daily graphic display for each of display items and for each of parts of the body at step S161 in Fig. 8B will be described in more detail with reference to Figs. 12 to 14. This process is to display the measurement data that is taken every day according to the flow chart for measurement in Figs. 6A and 6B and is stored in the memory unit 39, together with the date of measurement, on the display unit 7 in the form of bar-graph. In particular, when, after completion of measurement of body composition of the person under test, the measurement result is displayed on the display unit 7 according to the flow chart for process of display in Fig. 7, or the result of each type of measurement is numerically displayed on the display unit 7 according to the flow chart in Figs. 9 to 11, the graph key 21 in Fig. 2 may be turned ON. Then, it is determined that the answer of step S 154 is "Yes", and the graph counter is incremented from "0" to "1" at step S155. The routine proceeds to steps S160 and S161 and then to step S1000 in Fig. 12A to conduct the process of daily graphic display for each of display items and each of parts of body.

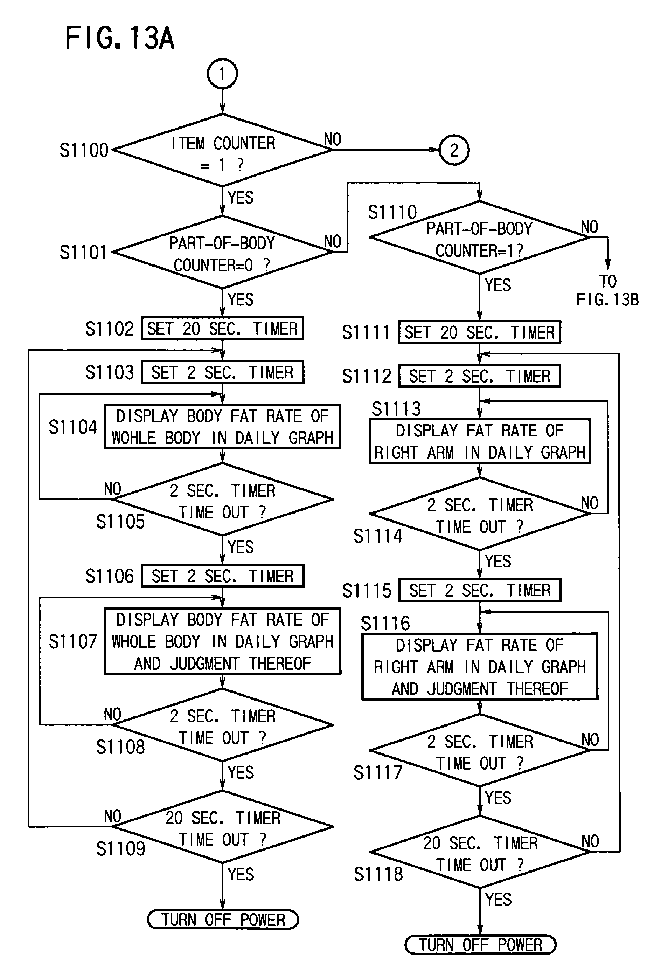

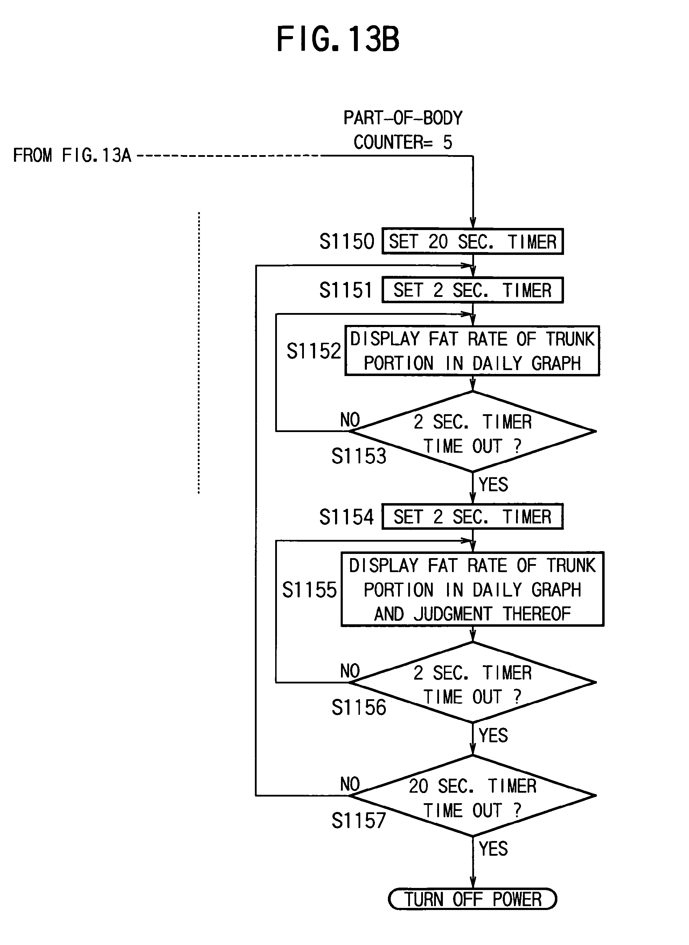

At step S1000 it is determined whether the item counter that is set in Fig. 8 is "0" or not. If it is "0" which means that the body weight is to be displayed then the routine proceeds to step S1001. However, if it is not "0" the answer thereof is "No" so that the routine proceeds to step S1100 in Fig. 13A.

At step S1001 it is determined whether the part-of-body counter that is set in Fig. 8 is "0" or not. If it is "0" which means that whole body is selected for the part of body then the routine proceeds to step S1002 where the 20 sec. timer is set for setting the time period during which display is made until the power is turned OFF. Then, at step S1003, the 2 sec. timer is set during which period the body weight of whole body is displayed in the form of daily graph at step S1004. In particular, at step S1004, the body weight of whole body that is measured at steps S105 to S107 in Fig. 6A and is stored in the memory unit 39 is displayed on the display unit 7 in the form of bar-graph including the body weight data for period of e.g. one month from the date of measurement to the past day of 30 days ago in the unit of a day, as shown in Fig. 24.

In this example, the characters representing the body weight of whole body, the date of measurement, and the numeral value of body weight are displayed, in addition to the bar-graph of body weight in the unit of a day with a star mark (*) positioned under the bar-graph for indicating the date of measurement. The star mark (*) may be moved in left or right-hand direction in response to depressing of the DOWN key 15 or UP key 16 in Fig. 2 for the purpose of changing or shifting the date of measurement. If the date of measurement is changed in this way then the date of measurement and the numerical value of the body weight displayed on the display unit are also changed accordingly. If there is no measurement data available on the date of measurement thus changed then a message "No Data Available" is displayed, instead of the body weight value. Changing the date of measurement with the DOWN key 15 or the UP key 16 is commonly applied to another process of graphic display, as described hereafter.

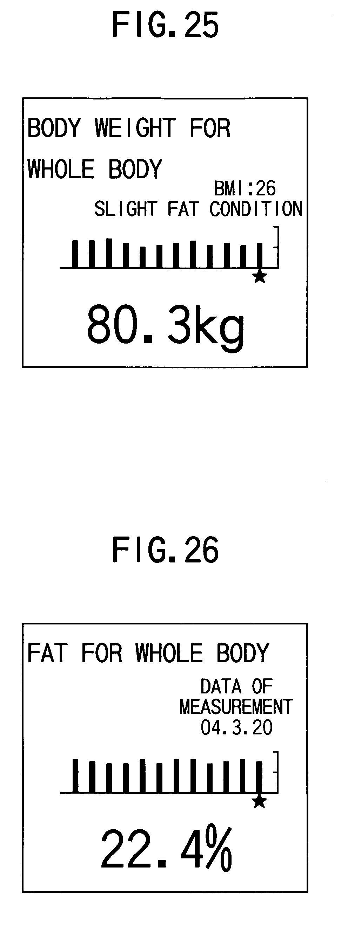

At step S1005 it is determined whether the 2 sec. timer is expired or not. If not, the routine returns to step S1004 where the body weight of whole body is graphically displayed. However, if it is expired, the routine proceeds to step S1006 where the 2 sec. timer is set again. At step S1007 the characters representing the body weight of whole body, the numeral value of body weight, the numerical value of body mass index BMI, and the judgment for adiposity based on the BMI, e.g. "Slightly Fat Condition" are displayed, together with the bar-graph including the body weight data for period of e.g. one month from the date of measurement to the past day of 30 days ago in the unit of a day with the star mark (*) for indicating the date of measurement, as shown in Fig. 25. In the same manner as above, the star mark (*) may be moved in left or right-hand direction in response to depressing of the DOWN key 15 or UP key 16. If the date of measurement is changed in such way then the date of measurement, the numerical values of the body weight and "BMI" as well as the judgment for adiposity displayed on the display unit are also changed accordingly.

At step S1008 it is determined whether the 2 sec. timer is expired or not. If so, the routine proceeds to step S1009. In this step S1009 it is determined whether the 20 sec. timer that has been set at step S1002 is expired or not. If not, the routine returns to step S1003 and the two types of display as described above are repeated. If they are repeated five times then the total time that has passed amounts to 20 sec., upon which it is determined that the timer is expired at step S1009, and as the result, the power is turned OFF.

If the part-of-body counter is "1", instead of "0", at step S1001 then the answer of step S1010 is "Yes". Then, the routine proceeds to step S1011 where the 20 sec. timer is set. Then, at step S1012, the weight of right arm that is stored in the memory unit 39 is retrieved and graphically displayed in the unit of a day, in the same manner as the body weight of whole body. Then, expiration of the timer is waited at step S1013, and the power is turned OFF. For weight of right arm its numerical value is simply displayed in this example because of no judgment criterion provided for classifying it into slender, standard, adiposity, etc. If in future some judgment criterion becomes available then two displays, i.e. graph of and judgment for weight of right arm can alternately be presented, as with the body weight of whole body at steps S1003 to S1008.

If the answer of step S1010 is "No" then the part-of-body counter may be any one of 2 to 5. In Fig. 12 branch paths for the case of the part-of-body counter = 2, 3, 4 and 5 are generally same, except for no decision block provided for the part-of-body counter = 5. Therefore, branch paths for the part-of-body counter = "2" and "3" and the corresponding descriptions are omitted, here.

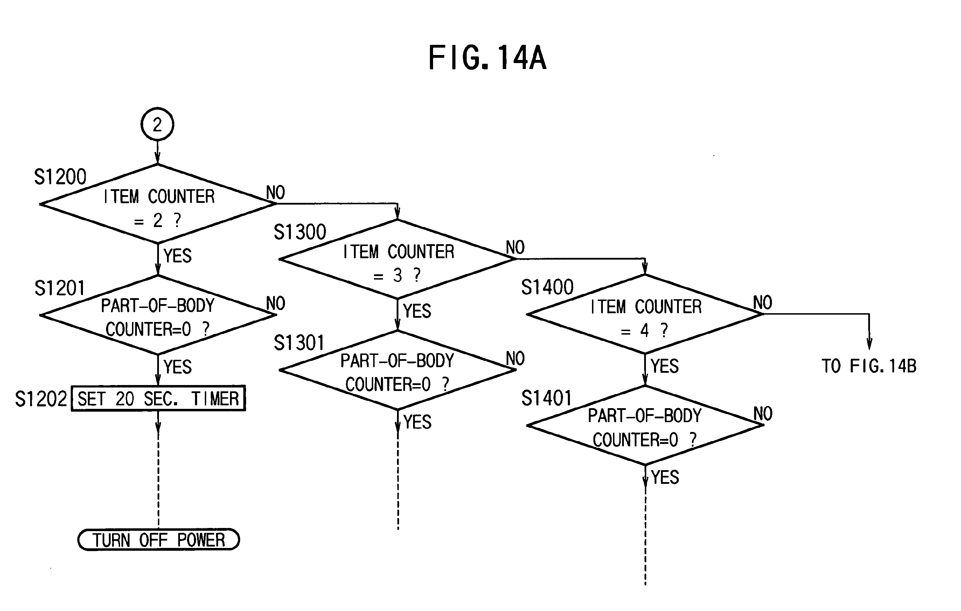

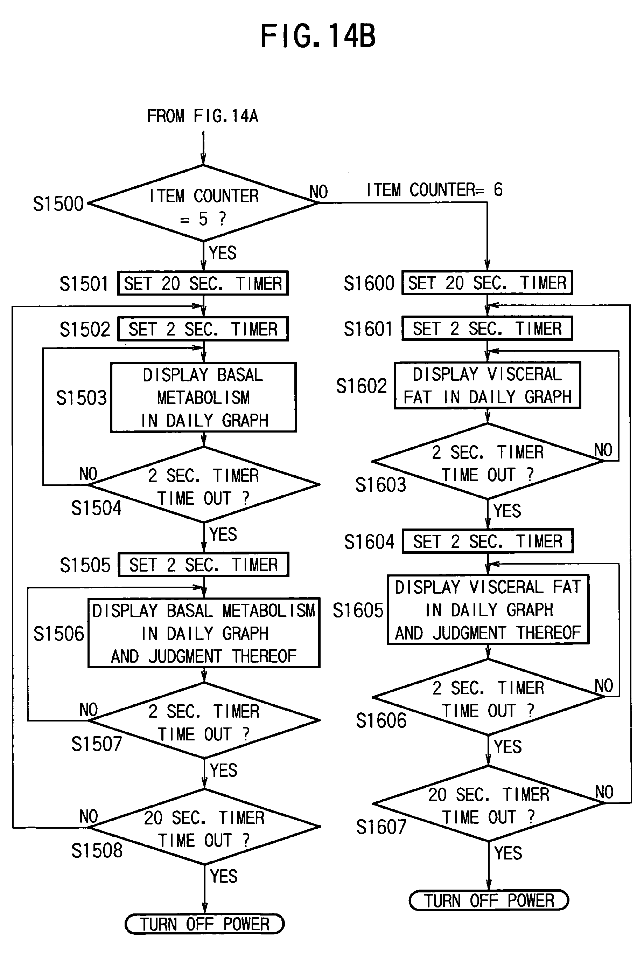

If the answer of step S1000 in Fig. 12A is "No" the routine proceeds to step S1100 in Fig. 13A. At step S1100 it is determined whether the item counter that is set in Fig. 8 is "1" or not. If so, the routine proceeds to step S1101 to display the body fat rate. However, if not, the answer is "No" so that the routine proceeds to step S1200 in Fig. 14A.

At step S1101 it is determined whether the part-of-body counter that is set in Fig. 8 is "0" or not. If it is "0" which means that whole body is selected for the part of body then the routine proceeds to step S1102 where the 20 sec. timer is set for setting the time period during which display is made until the power is turned OFF. Then, at step S1103, the 2 sec. timer is set during which period the body fat rate of whole body is graphically displayed at step S1104. In particular, at step S1104, the body fat rate of whole body that is stored in the memory unit 39 is graphically displayed in the unit of a day on the display unit 7, as shown in Fig. 26, in the same manner as the graphical display of body weight of whole body.

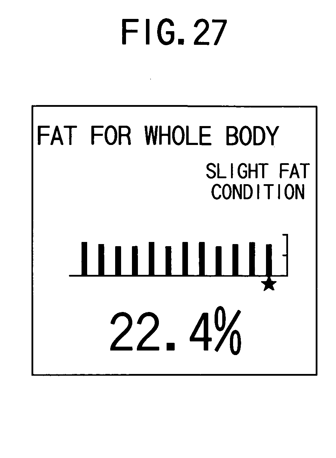

Then, at step S1105, it is determined whether the 2 sec. timer is expired or not. If the timer is not expired the routine returns to step S1104 where the body fat rate of whole body is graphically displayed in the unit of a day. However, if the timer is expired the routine proceeds to step S1106 where the 2 sec. timer is set again. Then, at step S1107, the characters representing the body fate rate of whole body and the numerical value thereof, and the judgment for adiposity, e.g. "Slightly Fat Condition", are displayed, together with the bar-graph including the data of the body fat rate of whole body in the unit of a day, as shown in Fig. 27. Then, at step S1108, it is determined whether the timer is expired or not. If so, the routine proceeds to step S1109. At this step S1109 it is determined whether the 20 sec. timer that has been set at step S1102 is expired or not. If not, the routine returns to step S1103 and the two types of display as described above are repeated. If they are repeated five times then the total time that has passed amounts to 20 sec., upon which it is determined that the timer is expired at step S1109, and as the result, the power is turned OFF.

If the part-of-body counter is "1", instead of "0", at step S1101 then the answer of step S1110 is "Yes". Then, the routine proceeds to step S1111 where the 20 sec. timer is set. Then, at step S1112, the 2 sec. timer is set during which period the fat rate of right arm is graphically displayed in the unit of a day at step S1113. In particular, at step S1113, the fat rate of right arm that is stored in the memory unit 39 is retrieved and graphically displayed, in the same manner as the body fat rate of whole body. Then, expiration of the timer is waited at step S1114, and then, the routine proceeds to step S 1115 where the 2 sec. timer is set during which period display is made at step S1116. At this step S1116 the fat rate of right arm and the result of judgment thereof stored in the memory unit 39 are displayed in the bar-graph including the data in the unit of a day. Expiration of the timer is waited at step S 1117, and then, the routine proceeds to step S1118. At this step S1118, it is determined whether the 20 sec. timer that has been set at step S1111 is expired or not. If not, the routine returns to step S1112 and the two types of display as described above are repeated. If they are repeated five times then the total time that has passed amounts to 20 sec., upon which it is determined that the timer is expired at step S1118, and as the result, the power is turned OFF.

If the answer of step S1110 is "No" then the part-of-body counter may indicate any one of 2 to 5. In Fig. 13 branch paths for the case of the part-of-body counter = 2, 3, 4 and 5 are generally same, except for display items. Therefore, branch paths for the part-of-body counter and the corresponding descriptions are partially omitted, here.

If the item counter is not "1" at step S1100 in Fig. 13A the answer thereof is "No" so that the routine proceeds to step S1200 in Fig. 14A. If the item counter is "2" at step S1200 then the routine proceeds to step S1201 and the subsequent steps because of display of muscle mass selected in Fig. 8. The flow chart representing such routine and the corresponding description are omitted, here, because the display item or body fat rate of the body fat rate graphical display process in Fig. 11 is only replaced with the muscle mass.

Furthermore, if it is determined that the item counter is "3" in step S1200 then the routine proceeds to step S1301. In this case, the display item is bone mass. In addition, if the item counter is "4" in step S 1400 then the routine proceeds to step S1401. In this case, the display item is body water mass. Moreover, if the item counter is "5" in step S1500 then the routine proceeds to step S1501. In this case, the display item is basal metabolism. Furthermore, if the item counter is "6" in step S1500 then the routine proceeds to step S1600. In this case, the display item is visceral fat area. The flow chart representing such routine and the corresponding description are omitted, here, because the display item in the case of process in Fig. 10 is only replaced with the relevant one.

In the process of daily graphic display for each of display items and each of parts of body, as shown in Figs. 12 to 14, if it is desired to provide an additional display for desired display item or desired part of body while one display is provided, then any relevant key selected among the display item selection key sub-group 19 and the part-of-body selection key sub-group 20 in Fig. 2 may be turned ON to cause a display key interruption process as shown in Fig. 8 so that the process of display in Figs 12 to 14 is executed to provide the desired display. That is to say, the person under test may simply turn the desired key ON to provide the desired display without any restriction to some operation procedure.

In the process of daily graphic display for each of display items and each of parts of body, if the graph key 21 is turned ON, then it is determined that the answer of the step S154 in Fig. 8B is "Yes". Then, the graph counter is incremented by 1 so that the routine proceeds to step S 163 where process of weekly graphic display for each of display items and each of parts of body is executed. For this process of weekly graphic display the flow chart representing such process and the corresponding description are omitted, here, because the daily data used in the graphic display in Figs. 12 to 14 is only replaced with the weekly data.

Furthermore, in the process of weekly graphic display for each of display items and each of parts of body, if the graph key 21 is turned ON, then it is determined that the answer of the step S 154 in Fig. 8B is "Yes". Then, the graph counter is incremented by 1 so that the routine proceeds to step S164 where process of monthly graphic display for each of display items and each of parts of body is executed. For this process of monthly graphic display the flow chart representing such process and the corresponding description are omitted, here, because the daily data used in the graphic display in Figs. 12 to 14 is only replaced with the monthly data.

The weekly data is prepared in such manner that the daily data is collected for a period of a week and is averaged over the days in the week, but except for the day(s) on which no measurement is made. Total data for 52 weeks or one year can be displayed for weekly data. In the same manner, the monthly data is prepared in such manner that the daily data is collected for a period of a month and is averaged over the days in the month, but except for the day(s) on which no measurement is made. Total data for 32 months or three years can be displayed for monthly data.

If any one key of the display selection key group 18 in Fig. 2 would be depressed during any process of numerical display, daily graphic display, weekly graphic display and monthly graphic display, each for each of display items and each of parts of body, then an interruption is caused to the microcomputer so that the interruption process according to the flow chart in Fig. 8 is executed to provide the display corresponding to the key depressed, as described above. Accordingly, it is very easy for the person under test to produce any desirable display.

The time period for the timer for continuing display, amount of data displayed on the display unit 7, etc. may be changed as necessary.

In the embodiment as above, all the numerical or graphic display has been produced on the basis of daily data. However, the present invention is not limited to such embodiment, but any modification thereof may be made. For example, the data acquired for specific time zone in a day, for example, for an hour of e.g. 9 o'clock or 20 o'clock, in AM or PM, in the morning, daytime or night, etc. may be stored in some memory unit from which only the relevant data may be retrieved for display.

In addition, the display item and the display format on the display unit 7 may be changed as necessary.

## Claims

1. A living body measurement apparatus comprising: a plurality of electrodes; a current supplying unit; a voltage measurement unit; a switching unit; a controller unit; and a display unit, wherein said plurality of electrodes are each contact with each of parts of a living body, said current supplying unit supplies electric current to the electrodes, said voltage measurement unit measures electric voltage on the electrodes, said switching unit switches between the electrodes so that the electrodes to which the current is supplied and on which the voltage is measured are changed, said controller unit controls operation of the current supplying unit, the voltage measurement unit and the switching unit, and said display unit is connected to the controller unit and displays the measurement result, said apparatus further comprising: a display item selection unit and a part-of-living body selection unit, whereby the measurement result selected by those two selection units is displayed on the display unit.

2. A living body measurement apparatus according to claim 1 in which it further comprises an additional selection unit which produces graphic display of the measurement result selected by said two selection units on the display unit.

3. Aliving body measurement apparatus according to claim 1 or 2 in which said measurement result is at least one of the followings: body weight; body fat rate; muscle mass; bone mass; body water mass; basal metabolism; and visceral fat area.
